# EUROPEAN PATENT SPECIFICATION

(11) **EP 2 322 177 B1**
(45) Date of publication and mention of the grant of the patent: **09.07.2014**
(21) Application number: 10190564.4
(22) Date of filing: 09.11.2010
(51) Int. Cl.: A61K 31/4422, A61P 15/02, A61P 21/02, A61K 9/00, A61K 31/23, A61K 31/232, A61K 31/4168, A61K 31/728, A61K 38/39, A61K 45/06

(54) **Use of a preparation based on nifedipine for treating vaginismus**
Verwendung einer Zusammensetzung aus Nifedipin zur Behandlung von Vaginismus
Utilisation d'une préparation contenant de la nifédipine pour traiter le vaginisme

(30) Priority: 12.11.2009 IT RM20090588
(43) Date of publication of application: 18.05.2011
(73) Proprietor: Antropoli, Carmine, 81020 Sant'Angelo in Formis, Capua CE (IT)
(72) Inventor: Antropoli, Carmine, 81020 Sant'Angelo in Formis, Capua CE (IT)
(74) Representative: Sarpi, Maurizio

(56) References cited:
- EP-A1- 1 269 999
- WO-A1-00/18374
- WO-A1-98/56323
- WO-A2-02/069906
- WO-A2-03/034987
- WO-A2-2005/034883
- WO-A2-2008/110872
- ANDERSSON K E ET AL: "Effects of nifedipine on myometrial activity and lower abdominal pain in women with primary dysmenorrhoea", BRITISH JOURNAL OF OBSTETRICS AND GYNAECOLOGY 1978 GB, vol. 85, no. 2, 1978, pages 142-148, XP8124556, ISSN: 0306-5456

## Description

### BRIEF SUMMARY OF THE INVENTION

The present invention relates to the topical use of a Nifedipine-based preparation for the treatment of urogenital disorders, such as vaginismus.

According to the invention, the local use of Nifedipine in synergistic association with one or more active principles and eudermic ingredients chosen in the group constituted by almond oil, hyaluronic acid, vitamin A (retinyl palmitate), allantoin and elastin, which increase the effectiveness and bioavailability thereof, acts positively on the pathological condition, relaxing the muscular tension and at the same time preventing onset of undesirable side effects.

### FIELD OF THE INVENTION

Urogenital disorders afflict a large number of women and have a profound effect on the quality of life in general. Even though a large number of preparations are present and available for the treatment of the majority of these pathological conditions, certain urogenital disorders, which cannot be put down exclusively to a physiopathological condition, such as vaginismus, in which the psychological component plays a crucial role, are still far from well understood.

Vaginismus is a psycho-physiological condition that disables a women from any type of vaginal penetration, including sexual penetration, insertion of tampons and penetrations during gynaecological examinations. It is characterized by an involuntary spasm of the muscle of the outer third of the vagina, in particular of the muscles of the perineum, of the vulva, and of the vaginal orifice; in the most serious cases of vaginismus, the spasm occurs before coitus, with hermetic closing of the lips of the vulva and of the vaginal ostium, rendering impossible the minimum contact with the vulvo-vaginal orifice.

A woman affected by vaginismus does not consciously control the spasm, and the muscular reflex that is triggered thereby can be compared to that of the eye that closes the eyelid when an object is directed towards it. The seriousness of vaginismus and the pain during penetration, including sexual penetration, varies from woman to woman.

Vaginismus can be divided into:
primary vaginismus: when a woman has never been able to undergo sexual penetration or any other type of penetration without experiencing pain. It can be easily identified in adolescents and women approaching twenty years of age, i.e., with the first attempts to use vaginal tampons, sexual experiences and first vaginal examinations, such as PAP-Test. Women affected by this type of vaginismus are not conscious thereof until the first attempt at having vaginal penetration. There are a series in factors, both organic and psychological, which can contribute to primary vaginismus:
   sexual abuse, rape, or attempt at sexual abuse;
   knowledge or witness of a sexual abuse at the expense of a third party, without being personally involved;
   domestic violence or conflict in the domestic environment;
   demonization of sex;
   sensation of pain linked to penetration, particularly linked to the concept of "breaking of the hymen" or to the concept of pain of the first sexual relationship;
   generalized anxiety;
   invasive physical trauma;
   fistulas consequent upon perineal lacerations not well cicatrized;
   herpes or various neglected and badly treated vaginal infections.

Secondary vaginismus: when a woman who has previously had vaginal penetrations develops vaginismus subsequently. This can be due to infections or trauma during infancy, or due to physiological causes.

Vaginismus in general can be classified, in relation to its level of seriousness, in four degrees:
- first degree: the spasm is localized in the pelvic lining and can be alleviated easily;
- second degree: the spasm is present in the pelvic lining but remains persistent;
- third degree: the patient raises her buttocks to avoid being examined by the gynaecologist;
- fourth degree: the most severe, the patient raises her buttocks, retracts and closes her thighs to prevent being examined.

The above classification, drawn up by Dr. Lamont, is currently in use with specialists and therapists.

Even though vaginismus is considered a rare pathological condition, in effect it represents a serious problem for numerous women in western countries but not only. Various studies have demonstrated how vaginismus affects from 12% to 17% of women under treatment at centres of sexual therapy and reaches in various countries, such as Morocco and Sweden, 6% of the population.

### THE TREATMENT

As mentioned above, there is a series in factors that can contribute to onset of vaginismus. There may be physiological and psychological causes, and the treatment required may depend upon the reason for which the woman has developed this pathological condition. Each case is different in itself, and an individual treatment is decidedly indicated and recommended.

There then exist psychological treatments, such as systemic desensitization, or physiological treatments, such as progressive vaginal dilation; progressive vaginal dilation involves the use of vaginal inserts, which progressively dilate the vagina and, having a progressively larger size, contribute to dilation and opening of the vagina itself.

In recent years, a new method of treatment of vaginismus has been developed, based upon the use of botulinum toxin, or preferably botulinum toxin of type A.

Botulinum toxin acts at the level of the peripheral nervous system, blocking release of acetylcholine by the vesicles of the neuro-muscular synapsis, causing a blockage of the muscular stimulation and hence a flaccid muscular paralysis.

The first studies that regarded the use of botulinum toxin for the treatment of vaginismus date back to 1997.

The real unsolved problem in this type of intervention is represented by the fact that the paralysis induced by the toxin is reversible in times that vary according to the muscle treated, the dose, the patient, and other unknown factors so that after 2-6 months from the treatment of skeletal terminals and after 8-12 months from the treatment of autonomic terminals, the patient must be treated again.

Furthermore, not all the patients affected by vaginismus can be treated with botulinum toxin; in fact, to be able to access the therapeutic protocol, there must be the certainty that the muscle in question, which surrounds the vagina and is contracted in women affected by vaginismus is effectively "hyperactive". Through a needle electromyograph it is possible to record the activity of the muscle. In general, it is hyperactive in 77.7% of women affected by severe vaginismus. Furthermore, by means of electromyography it has been possible to document the intensity and degree of muscular hyperactivity.

The psychological component should not be underestimated: the patient subjected to treatment with botulinum toxin must not be afraid of injections. The toxin is injected with a small needle at the level of the elevator muscle of the anus that surrounds the vagina and that is particularly contracted in patients affected by severe vaginismus. The dose is very low, because located close by are two very important sphincters, which regulate anal and urinary continence. An error in the dosage could involve incontinence, albeit temporary, but in any case troublesome.

A low dose on the other hand involves a certain constancy and repetitivity of the treatment, approximately every three months.

In certain cases, repeated treatment causes a slight desensitization, but the most serious problem occurs following upon the treatment of large muscles, which have to be treated with not particularly small amounts of neurotoxin. In these cases, there may occur an immunization with the production by the patient of type A anti-toxin antibodies with neutralizing action. Furthermore, certain rare subjects are resistant to neurobotulinum toxin type A from the start caused by the contraction of the orbicular muscle of the eyes.

In pharmacological treatment, alongside psychological and sexualogical therapy, the use of Nifedipine has recently been introduced in the treatment of vaginismus.

For instance in WO-A-02/069906, vaginismus is treated inter alia with calcium channel blockers (including nifedipine) and NO-donors such as nitroglycerine, especially with topical nitroglycerine.

Nifedipine is a known calcium-antagonist, of a dihydropiridine type, which, used via topical route, reduces contraction of the muscles responsible for the spasm.

Nifedipine in fact acts, at the muscular level, blocking the calcium channels, which perform a key role in the contraction of the sarcomere.

The muscular contraction, triggered by a nervous impulse, determines opening of the calcium channels, which are normally closed, located on the membranes of the sarcoplasmatic reticulum and hence exit of the ion with increase in the concentration of Ca++ in the cytoplasm of the muscle fibres. At high concentrations of Ca++, troponin binds the ion, causing displacement of tropomyosin (localized on the actin filament) from the sites allowing binding of myosin. The actin-myosin complex is formed, which activates cyclic contraction of the myosin bridges, enabling the cell to contract. Then, with the interruption of the nervous impulse, the calcium-dependent ATP-ase pump located on the membrane of the sarcoplasmatic reticulum rapidly removes the calcium from the cytoplasmatic site, thus arresting muscular contraction.

However, the use of Nifedipine by itself can give rise to a series of drawbacks. In fact, if the concentration of Nifedipine exceeds certain threshold values, it determines the onset of side effects, i.e., a whole series of hypersensitivity reactions, such as reddening of the skin, which can create serious problems in view of the area involved in the treatment. It is hence of critical importance to find the right concentration of Nifedipine that is able to act as well as possible as active principle, without causing onset of side effects. In this perspective, finding the right balance between a therapeutic dose of Nifedipine and its bioavailability represents a primary target for a Nifedipine-based formulation for the treatment of vaginismus.

The purpose of the present invention is to overcome the drawbacks and the limits of the antivaginismus products so far available, providing a valid alternative to botulinum toxin, and to preparations that envisage Nifedipine as single active principle, so as to be able to act as coadjuvant to the psychological therapy.

In fact, there has been proposed, as an alternative to botulinum toxin and to preparations based exclusively on Nifedipine, a new Nifedipine-based drug, in which present, in association, are other active principles with emollient and eudermic properties that act in synergy and as coadjuvant, and that enable reduction of the concentration of Nifedipine itself below the threshold of onset of untoward side effects.

In accordance with the present invention, there has thus been identified a formulation where the concentration of Nifedipine is reduced from a range of 0.3-10% to a range of 0.1-1%, preferably between 0.1 and 0.2%, at the same time improving the effectiveness in the treatment.

The active principles included in the formulation, in association with Nifedipine (0.1-1%), are:
almond oil
sodium hyaluronate
allantoin
elastin
vitamin A

In detail:
- almond (Prunus amygdalus) oil, can be contained in the formulation from 4% to 10%, preferably 5%, and has markedly emollient and hydrating properties, aiding and promoting regeneration of the skin. It performs a role of myorelaxant, which, united to its capacity for maintaining the vaginal mucosa lubricated, renders penetration simpler. It is moreover known to be involved in the prevention and reduction of stretch marks.
- sodium hyaluronate, or hyaluronic acid, can be contained in the formulation from 0.1% to 0.5 %, preferably 0.2%, and is a fundamental component of the derma. Thanks to its properties it bestows upon the skin elasticity and softness, has the capacity to penetrate the layers of the epidermis and to act within the skin, participating in the formation of collagen and connective tissue; hyaluronic acid protects the organism from viruses and bacteria, increases the plasticity of the tissues, and guarantees optimal cutaneous hydration. It moreover has cicatricial and antiinflammatory properties, and guarantees a correct lubrication.
- allantoin,: 0.1%, 2%, preferably 0.3%; it presents lenitive properties; being produced via the metabolic route referred to as "purine catabolism", it represents the primary route of transport of nitrogen in the organism. Thanks to its astringent and abrasive properties, it has the capacity to favour healing of minor wounds.
- elastin: 0.05% to 2%, preferably 0.1%; it presents elasticizing properties. It is a fundamental constituent of the skin and enables many tissues of the organism to return to their original form.
- vitamin A, or retinyl palmitate, can be contained in the formulation from 0.015% to 0.025%, preferably 0.015%. Vitamin A stimulates cell renewal and helps the skin to remain soft and hydrated, guaranteeing its protection.

All these active principles act together in improving the antivaginismus action of a galenic preparation, based upon Nifedipine, increasing in particular its effectiveness, the capacity of absorption and its bio-availability, and preventing onset of side effects such as reddening of the skin and cutaneous hypersensitivity, which factors of fundamental importance in view of the area involved.

Described hereinafter is the preparation of a formulation comprising the active principles according to the invention: it should be noted that this example is provided purely by way of illustration, and the present invention is not to be considered limited thereby, since this example is not meant to limit the formulation and the active principles.

### Formulation of a cream

**TABLE 1**

| NAME OF THE RAW MATERIAL | COMPOSITION % g/g | POSSIBLE FORMULATION |
|---|---|---|
| **Active Substances** | | |
| Nifedipine | 0.1 - 1.0 | 0.2 |
| Elastin | 0.05 - 2 | 0.1 |
| Allantoin | 0.1 - 2 | 0.3 |
| Vit. A (retinyl palmitate) | 0.015 - 0.025 | 0.015 |
| Sodium hyaluronate | 0.1 - 0.5 | 0.2 |
| Almond oil | 4.0 - 10.0 | 5.0 |

| **Excipients** | | |
|---|---|---|
| Cetomacrogol 1000 | | |
| Cetylstearylic alcohol | | |
| Glyceryl stearate SE | | |
| Isopropyl myristate | | |
| Lactic acid | | |
| Water | | |

| **Preservatives** | | |
|---|---|---|
| Imidazolidinyl urea | | |
| Bentylic alcohol | | |
| Methyl isothiazolinone | | |
| Methyl chloroisothiazolinone | | |

### EXPERIMENTAL RESULTS

The purpose of the present preliminary clinical study was to evaluate the effectiveness and tolerability of a treatment with the cream according to the formulation of Table 1, in women affected by vaginismus.

For the study, 40 women affected by vaginismus were recruited, under medical observation following upon gynaecological examination for onset of associated symptoms.

The total duration of the study was three months, and the study was conducted in two phases.

The first phase of the study covered a period of one month whilst the second phase a period of two months.

All the subjects agreed to sign consent for the use of said cream and to undergo checks of evaluation at 15 and 30 days.

The study protocol followed envisaged: choice of the population forming the subject of the study; a preliminary informative interview, complete with a general assessment of the psychological state; specialistic medical examinations for evaluation of the degree of vaginismus and the degree of effectiveness of the product.

The preliminary informative interview had a duration of approximately one hour and envisaged gleaning of anamnestic data, an illustration of the modalities and purposes of the study, as well as a psychological evaluation (it envisaged assessment of the degree of phobia present, assessment of other sexual dysfunctions, such as disturbance of desire, excitement or orgasm, co-existence of anxious-depressive disturbance).

The population of the study was constituted by 40 women affected by vaginismus aged between 19 and 49 (average 29.3), of whom 7 presented primary vaginismus, and 33 secondary vaginismus.

Of the 7 women affected by primary vaginismus only 4, after psychological consultancy, participated in the study. Of the other 3 excluded, 2 were affected by unperforated hymen and 1 was affected by perineum-vaginal infection and were referred, respectively, for surgical treatment and medical treatment.

Of the 33 women affected by secondary vaginismus, 8 were excluded since 4 were affected by infective processes (1 with herpes, 3 with badly treated vaginitis), 4 presented scars deriving from previous surgical operations and 1 was affected by serious disturbance of the psychological picture.

Consequently, 28 women participated in the study.

In the first medical examination, the following parameters were evaluated:
*Symptoms and signs*
*Psychological history* of the patient
*Type* of vaginismus (primary or secondary)
*Cause* of vaginismus (psychological or physical)
*Degree* of vaginismus (according to the classification proposed by Dr. Lamont)
Possible *previous treatments* (gradual desensitization, progressive dilations, botox injections, psychological therapy).

In the subsequent examinations the degree of vaginismus, and the penetration at the gynaecological examination were re-evaluated after daily application of the cream of Table 1.

### FIRST PHASE: First Examination

At the start of the study, all the women enrolled (28) were incapable of having a sexual relationship following upon onset of painful vaginal contractions. Five of the 28 related a story of conflict in the domestic environment. One reported previous episodes of sexual abuse during adolescence. The remaining subjects presented an apparent psychophysical balance. Four presented primary vaginismus and 24 secondary vaginismus. Having preliminarily excluded physical causes of vaginismus, all the patients were found to be affected by the psychological form. According to the clinical classification proposed by Dr. Lamont, the patients were classified as follows: 5 presented a first-degree vaginismus; 15 presented a second-degree vaginismus; 6 a third-degree vaginismus; and 2 a fourth-degree vaginismus.

Of the entire population, only two patients reported having undergone treatment by means of progressive dilations, obtaining poor resolution of the painful symptoms. All the women received a sample of cream according to the present invention, namely a compound of Nifedipine-based galenic formulation, emollients, and eudermic compound, as already described.

The women were recommended to apply it at the level of the vulvo-vaginal ostium every evening and to attend the subsequent pre-set checks (at 15 and 30 days).

**TABLE 2**

| Degree acc. to Lamont | |
|---|---|
| 1st | 5 (17.9%) |
| 2nd | 15 (53.6%) |
| 3rd | 6 (21,40) |
| 4th | 2 (7.1%) |
| Total | 28 |

### FIRST PHASE: EVALUATION AT THE SECOND EXAMINATION

All the patients were re-assessed after 15 days from the first application of the cream according to the invention.

The women were questioned on the individual compliance to the treatment prescribed, directing attention to the reported appearance of local and/or systemic adverse events.

No subject reported intolerance to the product; only two patients deemed that the modalities of the treatment prescribed was not very compatible with their own day-to-day habits, interfering in their life style.

During the gynaecological examination, the degree of vaginismus according to Dr. Lamont was re-assessed in all the patients. Of the 28 women, 3 (10.7%) presented an improvement in their clinical picture: 2 patients with a vaginismus initially classified as of second degree now had first-degree vaginismus; whereas one who originally had third-degree vaginismus, now showed an improvement such as to be included in the second degree, as emerges from Table 3 below.

**TABLE 3**

| Degree acc. to Lamont | |
|---|---|
| 1st | 7 (25%) |
| 2nd | 14 (50%) |
| 3rd | 5 (17,9%) |
| 4th | 2 (7.1%) |
| Total | 28 |

### FIRST PHASE: THIRD EXAMINATION

All patients were re-evaluated after 30 days from the first application of the cream.

At the anamnestic interview, no subject reported onset of new adverse events with respect to the last check. A patient classified in the study as affected by first-degree vaginismus reported having attempted a sexual relationship, obtaining a good result.

The re-evaluation of the degree of vaginismus according to Lamont at the gynaecological examination evidenced: 8 women with a complete resolution of vaginismus (one of whom was the patient that spontaneously had attempted a sexual relationship); of these 7 passed from a first degree of the second examination to resolution; and 1 passed directly from a second degree of the second examination to resolution of the pathological condition.

Ten second-degree patients at the second examination improved to the first degree, two patients passed from the third degree to the second degree, whereas one patient passed from the fourth degree to the third degree.

Five (17.9 %) patients did not show an improvement of the degree of vaginismus after 30 days of applications of the cream, whilst as many as 23 (82.1%) patients out of 28 showed after a month of daily treatment an improvement of their degree of vaginismus.

**TABLE 4**

| Degree acc. to Lamont | |
|---|---|
| Resolution | 8 (28,6%) |
| 1st | 11 (39,3%) |
| 2nd | 4 (14.3%) |
| 3rd | 4 (14,3%) 15 |
| 4th | 1 (3.5%) |
| Total | 28 |

The result of the different examinations for each patient are compared in Table 5 below.

**TABLE 5**

| | **First** **examination** | **Second** **examination** | **Third** **examination** |
|---|---|---|---|
| **Pat. 1** | 2nd | 2nd | 1^{st} |
| **Pat. 2** | 1st | 1st | RESOL |
| **Pat. 3** | 3rd | 3rd | 2^{nd} |
| **Pat. 4** | 2nd | 2nd | 1^{st} |
| **Pat. 5** | 4th | 4th | 4^{th} |
| **Pat. 6** | 3rd | 3rd | 2^{nd} |
| **Pat. 7** | 2nd | 1st | RESOL |
| **Pat. 8** | 1st | 1st | RESOL |
| **Pat. 9** | 2nd | 2nd | 1st |
| **Pat.10** | 3rd | 2nd | 3rd |
| **Pat. 11** | 2nd | 1st | RESOL |
| **Pat. 12** | 2nd | 2nd | 1st |
| **Pat. 13** | 3rd | 3rd | 3rd |
| **Pat. 14** | 2nd | 2nd | 1st |
| **Pat. 15** | 1st | 1st | RESOL |
| **Pat. 16** | 2nd | 2nd | 1st |
| **Pat. 17** | 3rd | 3rd | 3rd |
| **Pat. 18** | 4th | 4th | 3rd |
| **Pat. 19** | 2nd | 2nd | 1st |
| **Pat. 20** | 1st | 1st | RESOL |
| **Pat. 21** | 2nd | 2nd | 2nd |
| **Pat. 22** | 2nd | 2nd | RESOL |
| **Pat. 23** | 2nd | 2nd | 1st |
| **Pat. 24** | 2nd | 2nd | 1st |
| **Pat. 25** | 2nd | 2nd | 1st |
| **Pat. 26** | 1st | 1st | RESOL |
| **Pat. 27** | 3rd | 3rd | 3rd |
| **Pat. 28** | 2nd | 2nd | 1st |

### SECOND PHASE OF THE STUDY

In the second phase of the study, exclusively the patients who presented at the end of the first phase of the study a degree of vaginismus that was very slight to slight were enrolled.

The population of the study consisted of 19 patients, i.e., the ones that at the end of the first phase of the study presented first degree of vaginismus (11 women) and the 8 women that had presented a resolution of vaginismus at the gynaecological examination.

This phase envisaged the application of a new formulation of cream according to the invention, constituted by a higher percentage of Nifedipine and lubricating substances, and which had to be applied half an hour before each sexual relationship at the level of the vulvo-vaginal ostium.

After two months, all the patients were re-evaluated with a gynaecological examination and gleaning of data on the effectiveness and reliability of the product.

All the women, in the period of the two months, declared having had at least one sexual relationship, on average 5.1 relationships (range 1-12). No subject experienced intolerance and/or untoward reactions to the product.

Fifteen patients declared having had relationships progressively less painful, even pleasurable relationships.

The gynaecological examination documented a good penetration at the speculum, with the absence of involuntary contractions of the perineal muscles that surround the outer third of the vagina.

### Conclusions

The result of our study have highlighted a high effectiveness of the cream forming the subject of the invention in the treatment of both primary and secondary vaginismus.

The product proved effective in reducing the degree of vaginismus in 82.1% of the patients after daily application for a month; whilst a pre-coitus use of the cream represented a surprising aid for women affected by very slight-to-slight vaginismus, enabling even pleasurable relationships.

In particular, from the results obtained in these studies, it may be suggested that the pharmacological approach with the Nifedipine-based drug, with synergistic action of different active principles such as almond oil, elastin, allantoin, vitamin A, and hyaluronic acid, is able to interrupt the vicious cycle "anxiety-fear of penetration-contraction-pain".

In synergy with psychological and sexualogical therapy it represents a new form of treatment for those cases that were previously deemed "untreatable", preventing and avoiding recourse to the costly and more invasive inoculations of botulinum toxin, moreover adding the simplicity of home use.

## Claims

1. Use of Nifedipine in association with one or more eudermic active principles chosen in the group of: almond oil, hyaluronic acid, vitamin A, elastin, and allantoin, for the preparation of a product for topical treatment of vaginismus.

2. Use of Nifedipine in association with one or more eudermic active principles as per Claim 1 for the preparation of a product for the topical treatment of vaginismus, wherein Nifedipine is present in the amount of 0.1-1.0 wt% of the product itself, preferably between 0.1 wt% and 0.2 wt%.

3. A pharmaceutical composition for use in the topical treatment of vaginismus, comprising:
Nifedipine: between 0.1 and 1 wt% over the total weight,
almond oil: between 4.0 and 10 wt%
sodium hyaluronate: between 0.1 and 0.5 wt%
allantoin: between 0.1 and 2 wt%
elastin: between 0.05 and 2%
vitamin A: between 0.015 and 0.025 wt%
in addition to excipients and preservatives.

4. A pharmaceutical composition for use in the topical treatment of vaginismus according to the preceding claim, wherein the excipients are comprised in the group: Cetomacrogol 1000, cetylstearylic alcohol, glycyl stearate SE, isopropyl myristate, lactic acid, and water.

5. The pharmaceutical composition for use as per the preceding claim, wherein the preservatives are comprised in the group: imidazolidinyl urea, benzyl alcohol, methyl isothiazolinone, and methyl chloroisothiazolinone.

6. The pharmaceutical composition for use as per Claims 3 onwards, wherein the concentration of Nifedipine is 0.2 wt% over the total weight.

7. The pharmaceutical composition for use as per Claims 3 onwards, **characterized in that** it is in a form selected from the group comprising gel, ointment, cream, lotion, powder, solution, suspension, spray, paste, oil, suppository, and foam.

## Patentansprüche

1. Verwendung von Nifedipin in Verbindung mit einem oder mehreren eudermischen aktiven Grundbestandteilen, ausgewählt in der Gruppe von: Bittermandelöl, Hyaluronsäure, Vitamin A, Elastin und Allantoin, für die Zubereitung eines Produkts zur lokalen Behandlung von Vaginismus.

2. Verwendung von Nifedipin in Verbindung mit einem oder mehreren eudermischen aktiven Grundbestandteilen nach Anspruch 1 für die Zubereitung eines Produkts zur lokalen Behandlung von Vaginismus, wobei Nifedipin in der Menge von 0,1 - 1,0 Gew.-% des Produktes selbst, vorzugsweise zwischen 0,1 Gew.-% und 0,2 Gew.-%, vorhanden ist.

3. Arzneimittelzusammensetzung zur Verwendung bei der lokalen Behandlung von Vaginismus, umfassend:
Nifedipin: zwischen 0,1 und 1 Gew.-% über dem Gesamtgewicht,
Bittermandelöl: zwischen 4,0 und 10 Gew.-%,
Natriumhyaluronat: zwischen 0,1 und 0,5 Gew.-%;
Allantoin: zwischen 0,1 und 2 Gew.-%,
Elastin: zwischen 0,05 und 2 %,
Vitamin A: zwischen 0,015 und 0,025 Gew.-%
zusätzlich zu Arzneimittelträgern und Konservierungsmitteln.

4. Arzneimittelzusammensetzung zur Verwendung bei der lokalen Behandlung von Vaginismus nach dem vorhergehenden Anspruch, bei der die Arzneimittelträger in der Gruppe: Cetomacrogol 1000, Cetylstearylalkohol, Glycylstearat SE, Isopropylmyristat, Milchsäure und Wasser enthalten sind.

5. Arzneimittelzusammensetzung zur Verwendung nach dem vorhergehenden Anspruch, bei der die Konservierungsmittel in der Gruppe: Imidazolidinylharnstoff, Benzylalkohol, Methylisothiazolinon und Chlormethylisothiazolinon enthalten sind.

6. Arzneimittelzusammensetzung zur Verwendung nach Ansprüchen 3 weiter, bei der die Konzentration von Nifedipin 0,2 Gew.-% über dem Gesamtgewicht ist.

7. Arzneimittelzusammensetzung zur Verwendung nach Ansprüchen 3 weiter, **dadurch gekennzeichnet, dass** sie in einer Form vorliegt, die aus der Gel, Salbe, Creme, Lotion, Puder, Behandlungslösung, Suspension, Spray, Paste, Öl, Zäpfchen und Schaum umfassenden Gruppe ausgewählt ist.

## Revendications

1. Utilisation de la Nifédipine en association avec un ou plusieurs principes actifs eudermiques dans le groupe comprenant : huile d'amande, acide hyaluronique, vitamine A, élastine et allantoïne, pour la préparation d'un produit pour le traitement tipique du vaginisme.

2. Utilisation de la Nifédipine en association avec un ou plusieurs principes actifs eudermiques selon la revendication 1, pour la préparation d'un produit pour le traitement topique du vaginisme, dans laquelle la Nifédipine est présente dans la quantité de 0,1-1,0 % en poids du produit lui-même, de préférence entre 0,1 % en poids et 0,2 % en poids.

3. Composition pharmaceutique pour l'utilisation dans le traitement topique du vaginisme, comprenant :
Nifédipine : entre 0,1 et 0,5 % en poids sur le poids total,
huile d'amande : entre 4,0 et 10 % en poids
hyaluronate de sodium :entre 0,1 et 0,5 % en poids
allantoïne : entre 0,1 et 2 % en poids
élastine : entre 0,05 et 2 % en poids
vitamine A : entre 0,015 et 0,025 % en poids
en plus d'excipients et de conservateurs.

4. Composition pharmaceutique pour l'utilisation dans le traitement topique du vaginisme selon la revendication précédente, dans laquelle les excipients sont compris dans le groupe : Cetomacrogol 1000, alcool cétylstéarylique, stéarate de glycyle SE, myristate d'isopropyle, acide lactique et eau.

5. Composition pharmaceutique pour l'utilisation selon la revendication précédente, dans laquelle les conservateurs sont compris dans le groupe : imidazolidinyl urée, alcool benzylique, méthylisothiazolinone et méthylchloroisothiazolinone.

6. Composition pharmaceutique pour l'utilisation selon la revendication 3 et les suivantes, dans laquelle la concentration de la Nifédipine est de 0,2 % en poids sur le poids total.

7. Composition pharmaceutique pour l'utilisation selon la revendication 3 et les suivantes, **caractérisée en ce qu'**elle est sous une forme sélectionnée dans le groupe comprenant : gel, onguent, crème, lotion, poudre, solution, suspension, pulvérisation, pâte, huile, suppositoire et mousse.
